# EUROPEAN PATENT APPLICATION

(11) **EP 4 344 714 A1**
(43) Date of publication of application: **03.04.2024**
(21) Application number: 23198747.0
(22) Date of filing: 21.09.2023
(51) Int. Cl.: A61M 5/165, A61M 39/10, A61M 5/38

(54) **FILTER FOR INFUSION MEDICAL LINES**

(30) Priority: 28.09.2022 IT 202200019893
(71) Applicant: Industrie Borla S.p.A., 10024 Moncalieri (Torino) (IT)
(72) Inventor: GUALA, Gianni, 10133 Torino (IT)
(74) Representative: Buzzi, Franco

(57) **Abstract**

Filter (1) for infusion medical lines comprising a box-like body (2) within which there are arranged four filtering hydrophilic membranes (16A,16B,16C,16D) parallel and spaced from each other so as to form three interspaces (23A,23B,23C) placed in communication with the inlet connector (9) of the liquid to be filtered.

## Description

### Field of the invention

The present invention regards a filter for infusion medical lines and more in particular an IV filter.

Such filters conventionally comprise a flattened box-like body having a first end wall and a second end wall, a pair of greater lateral walls, and a pair of smaller lateral walls. These filters are provided with an inlet connector for a liquid to be filtered and an outlet connector for the filtered liquid at the one and the other end wall. Arranged in the box-like body is a plate-shaped element formed on whose at least one of the opposite faces is a plurality of ribs defining parallel channels. Arranged on the or on each plurality of ribs is a respective filtering hydrophilic membrane which separates the channels from one or from respective interspaces delimited by the two greater walls. The or each interspace is placed in communication with the inlet connector and the channels are placed in communication with the outlet connector. Furthermore, the or each interspace is placed in communication with the external by means of openings with associated hydrophobic membranes for deaerating the liquid.

### State of the prior art

Document WO2019215516 owned by the same Applicant discloses a filter thus made which provides for a pair of interspaces for the through-flow of the liquid inside a box-like body provided with a plate-like element on whose faces there are arranged a pair of filtering hydrophilic membranes.

Documents EP1208857, EP1421960, US4525182 and US5439587 also disclose such filters, each comprising one or a pair of hydrophylic membranes to filter the liquid which flows from one or a pair of respective grooves delimited by the box-like body of the filter.

The main drawback of such configuration of the filtration path, especially in medical lines which require a high liquid filtration flow rate, stems from the fact that the two hydrophylic membranes do not always guarantee a sufficient flow rate for the filtered liquid. Therefore, in order to overcome this problem, there arises the need to significantly increase the dimensions - in plan view - of the filters to house membranes with larger filtering surfaces, jeopardising the ease of use making such prior art filters cumbersome and difficult to handle for the health personnel.

Furthermore, due to the high flow rates of the fluid to be filtered, the systems for the deaeration of the aforementioned prior art filters do not always guarantee a full deaeration of the entire liquid, with the disadvantage lying in the fact that the filter loses filtration efficiency given that when the membrane is in contact with air, it does not let the liquid flow through.

For example, document DE3005229A1 discloses a filter for medical lines of the type defined above and corresponding to the pre-characterising part of claim 1, in which the second interspace, or intermediate interspace, is insulated with respect to the external and it is placed in communication with the first and the third interspace at the end thereof facing towards the inlet connector. Despite being appropriate to guarantee a discrete flow rate of the filtered liquid, this arrangement has a filtering capacity limited by the insufficient deaeration of the liquid present in the central section of the filter.

### Summary of the invention

The object of the present invention is to overcome the aforementioned drawbacks.

With the aim of attaining such object, the invention provides a filter for infusion medical lines as defined in claim 1.

Such characteristics allow the filter according to the invention to double both the flow rate of the filtered liquid and the deaeration capacity while maintaining the same surface area - in plan view - with respect to the prior art filters. Furthermore, the arrangement of the vent openings allows to carry out the priming of the circuit in all positions of the filter.

In a preferred embodiment of the filter according to the invention, at the ends of the opposite faces of each of the plate-like elements there are provided for respective transverse manifolds on which there are arranged the hydrophobic membranes, the transverse manifolds are placed in communication with the vent openings.

The inlet and outlet connectors for the liquid can be designed for tube-tube or luer-tube or tube-luer or luer-luer connections of the medical line.

Each filtering hydrophilic membrane can also be obtained as a single piece with the respective hydrophobic membranes.

### Brief description of the drawings

Further characteristics of the invention will be apparent from the detailed description that follows, with reference to the attached drawings, provided purely by way of non-limiting example, wherein:
- figure 1 is a schematic perspective view of an embodiment of the filter according to the invention,
- figure 2 is a plan and partially cross-sectional view of figure 1,
- figure 3 is a front elevational view of figure 1,
- figure 4 is an exploded view of the filter of figure 1,
- figure 5 is a longitudinal cross-sectional view according to line A-A of figure 2,
- figure 6 is an enlargement of a portion the filter of figure 5,
- figure 7 is a cross-sectional view according to line B-B of figure 2,
- figure 8 is a cross-sectional view according to line D-D of figure 2
- figure 9 is a longitudinal cross-sectional view according to line F- F of figure 2.

### Detailed description of the invention

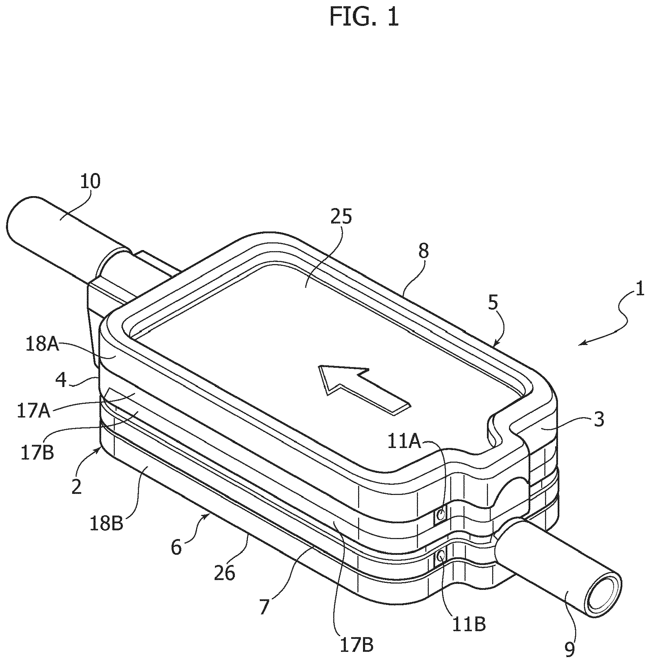

Initially with reference to figures 1-3, the infusion filter according to the invention is indicated in its entirety with 1.

It comprises a box-like body 2 formed by a pair of half-shells 5, 6. The box-like body 2 comprises a first and a second end wall 3, 4, a pair of larger side walls 25, 26, and a pair of smaller side walls 7, 8. At the first end wall 3 there is arranged an inlet connector 9 for a liquid to be filtered and at the second end wall 4, consisting of a hollow insert, there is arranged an outlet connector 10 for the filtered liquid.

A first vent opening, in the form of a hole 11A, is provided for in an upper portion of the first end wall 3 in proximity of the inlet connector 9, and a second vent opening, in the form of a hole 11B, is provided in a lower portion of the first end wall 3 in proximity of the inlet connector 9. A third vent opening, in the form of a hole 11C, is provided for in an upper portion of the second end wall 4 in proximity of the outlet connector 10, and a fourth vent opening, in the form of a hole 11D, is provided for in a lower portion of the second end wall 4 in proximity of the outlet connector 10.

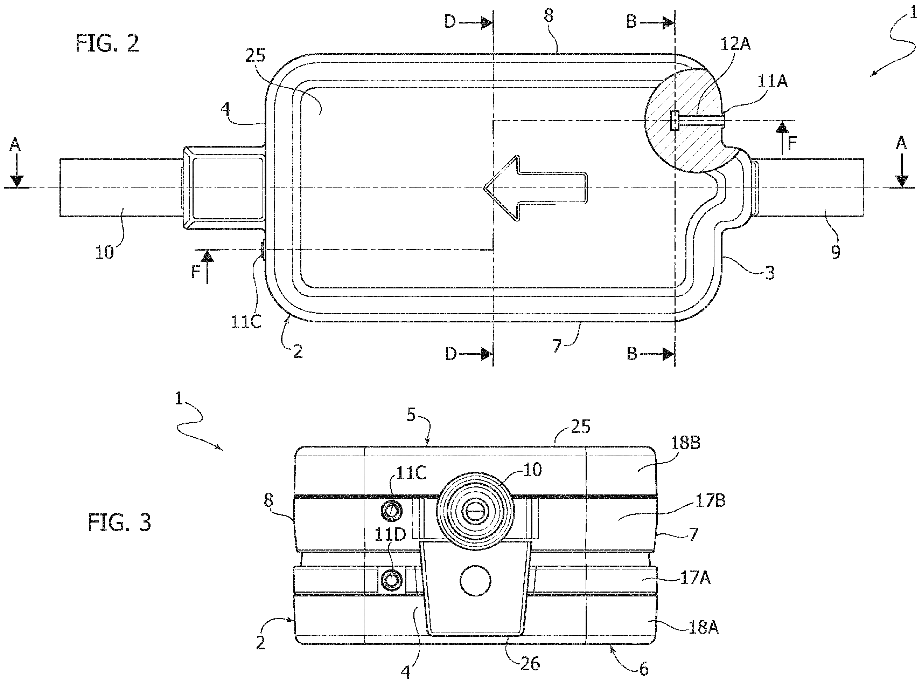

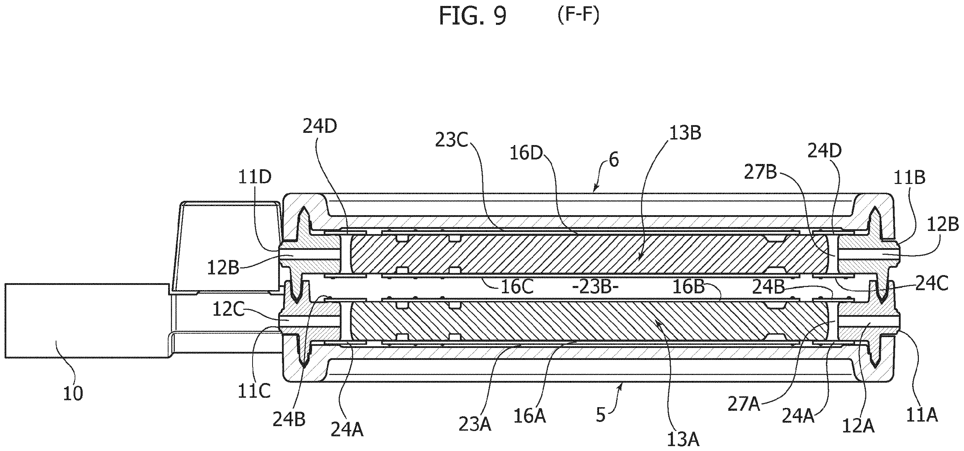

Visible in the partial section of figure 2 is a duct 12A which connects the hole 11A with the internal of the box-like body 2. Similar ducts 12B, 12C, 12D visible in figure 9, respectively connect the holes 11B,11C,11D with the internal of the box-like body 2 as mentioned hereinafter.

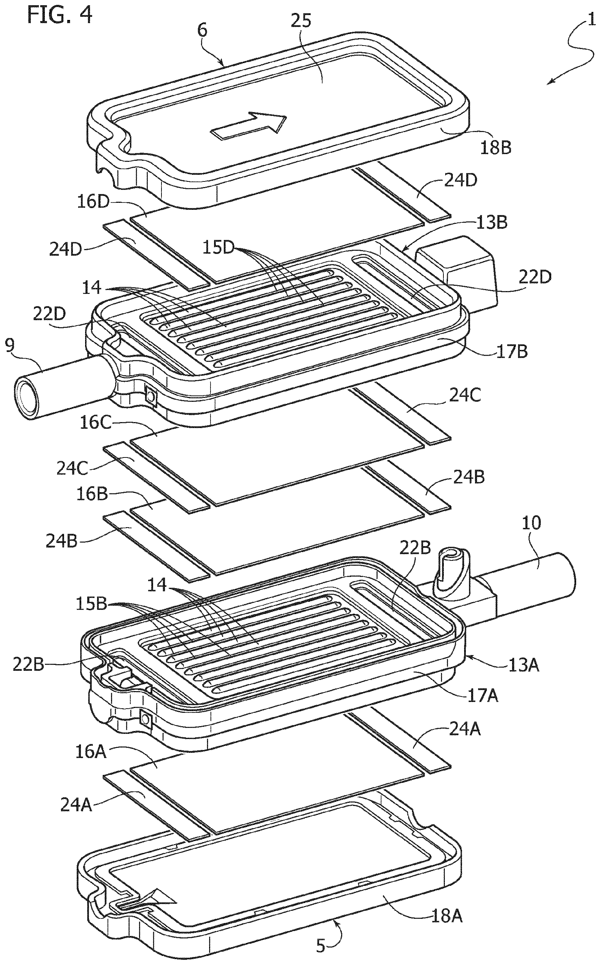

Now, with reference to figures 4 to 8, the first plate-like element in the half-shells 5, 6 on whose opposite faces there are formed respective pluralities of ribs 14 which define the channels 15A and 15B which extend longitudinally between the connectors 9, 10, is indicated with 13A.

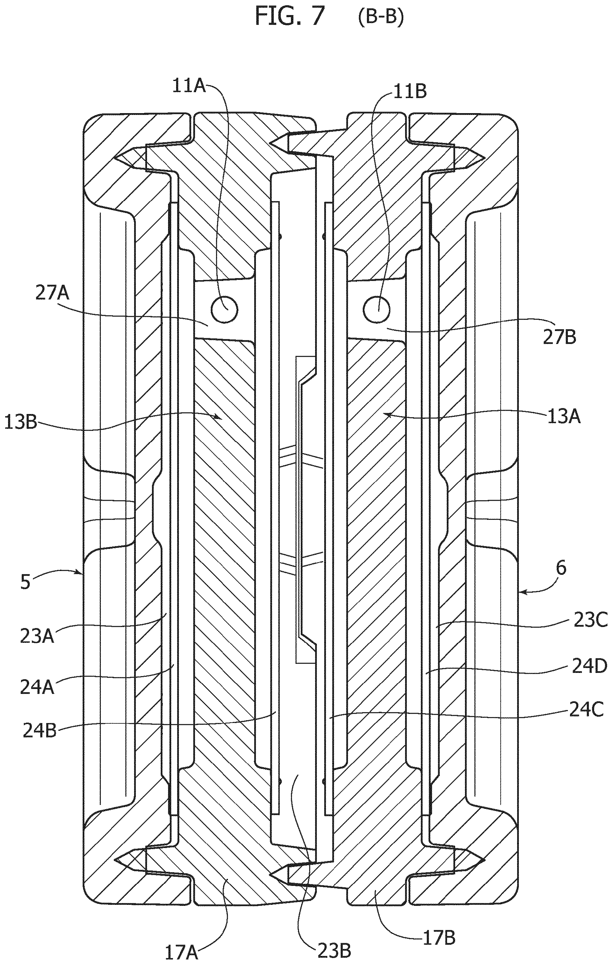

In proximity of both ends of the channels 15A, 15B of each face and perpendicularly thereto there are provided for respective transverse manifolds 22A-22B. The opposite manifolds 22A-22B of each end of the plate-like element 13A are placed in communication with each other through respective passages of the plate-like element 13A, one of which is indicated with 27A in figure 7, and furthermore they are placed in communication respectively with the vent hole 11A and the vent hole 11C.

Resting on each face of the plate-like element 13A is a respective hydrophilic membrane 16A-16B which is welded to the plate-like element 13A. Two pairs of hydrophobic membranes 24A-24B are arranged above the four manifolds 22A-22B and fixed, for example by welding. The two pairs of hydrophobic membranes 24A-24B can also be integrated and obtained as a single piece respectively with the one and with the other hydrophilic membrane 16A-16B.

The plate-like element 13A has a protruding perimeter edge 17A which is coupled - at the upper part - with annular lips 18A of the half-shell 5.

Still with reference to figures 4 to 8, the second plate-like element in the half-shells 5, 6 on whose opposite faces there are formed respective pluralities of ribs 14 which define the channels 15C and 15D which extend longitudinally between the connectors 9, 10, is indicated with 13B.

In proximity of both ends of the channels 15C, 15D of each face and perpendicularly thereto there are provided for respective transverse manifolds 22C-22D. The opposite manifolds 22C-22D of each end of the plate-like element 13B are placed in communication with each other through respective passages of the plate-like element 13B, one of which is indicated with 27B in figure 7, and furthermore they are placed in communication respectively with the vent hole 11B and the vent hole 11D.

Resting on each face of the plate-like element 13B is a respective hydrophilic membrane 16C-16D which is welded to the plate-like element 13B. Two pairs of hydrophobic membranes 24C-24D are arranged above the four manifolds 22C-22D and fixed, for example by welding. The two pairs of hydrophobic membranes 24C-24D can also be integrated with the one and with the other hydrophilic membrane 16C-16D.

The plate-like element 13B has a protruding perimeter edge 17B which is coupled - at the lower part - with respective annular lips 18B of the half-shell 6 and - at the top part - with the protruding perimeter edge 17A of the plate-like element 13A.

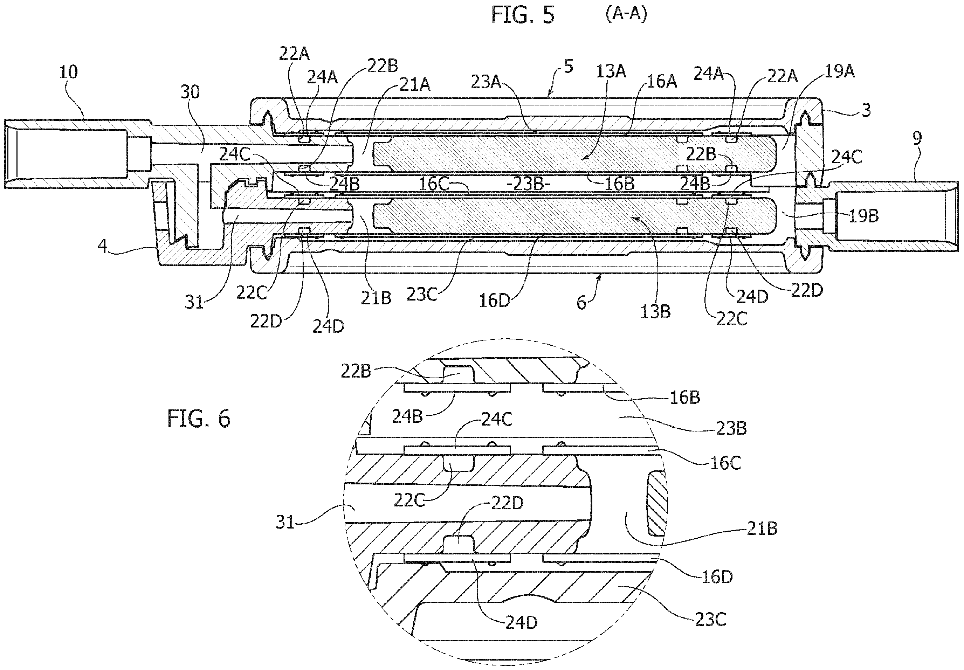

Figures 5-8 respectively show: a first interspace 23A, coplanar to the upper face of the plate-like element 13A, separated therefrom by means of the hydrophilic membrane 16A and delimited - at the upper part - by the half-shell 5, a second interspace or intermediate interspace 23B delimited - at the upper part - by the hydrophilic membrane 16B and - at the lower part - by the hydrophilic membrane 16C, and a third interspace 23C, coplanar to the lower face of the plate-like element 13B, separated therefrom by means of the hydrophilic membrane 16D and delimited - at the lower part - by the half-shell 6.

A passage 19B, shown in figure 5, provided for on the plate-like element 13B at the end of the inlet connector 9 places in communication the interspaces 23B, 23C with such connector 9.

A passage 19A provided for on the plate-like element 13A at the end of the inlet connector 9 places in communication the interspace 23A with the passage 19B.

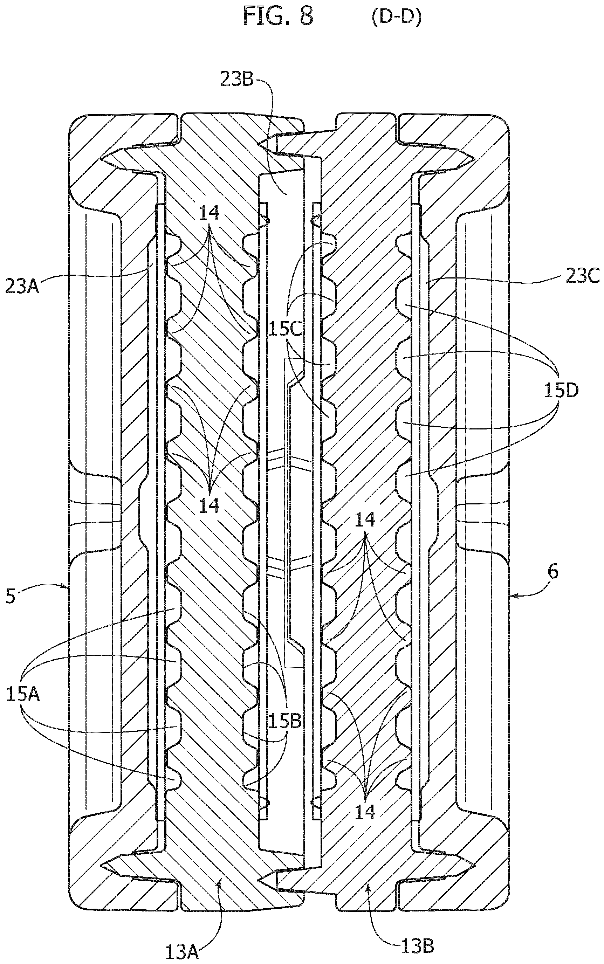

With reference to figure 5, a passage 21A of the plate-like element 13A places in communication the outlet connector 10 with the plurality of channels 15A, 15B, shown in figure 8, by means of the duct 30, also formed on the plate-like element 13A. A passage 21B of the plate-like element 13B places in communication the outlet connector 10 with the plurality of channels 15C, 15D.

Still with reference to figure 5, the outlet connector 10 comprises a double insert sealingly fixed to the box-like body 2 and forming a first duct 30 placed in communication with the interspaces 23A and 23B through the passage 21A, and a second duct 31 placed in communication with the interspaces 23B and 23C through the passage 21B. In this manner, the flows of the filtered liquid through the hydrophylic membranes 16A, 16B - on one side - and 16C, 16D - on the other hand - are separated through ducts 30, 31 until the outflow from the connector 10.

As it will be clear from the above, the filter 1 according to the invention has a compact and small configuration despite doubling the flow rate of the filtered liquid with respect to a similar filter of the prior art.

During use, the infusion liquid - through the inlet connector 9 and the duct 19B and 19A - respectively flows into the three interspaces 23A, 23B and 23C initially touching the proximal hydrophobic membranes 24A, 24B, 24C, 24D which allow the air trapped in the liquid to flow out into the respective manifolds 22A, 22B, 22C, 22D before being dispersed outside the filter 1 through the respective ducts 12A, 12B and the holes 11A, 11B. Subsequently, the fluid touches and flows through the filtering hydrophilic membranes 16A, 16B, 16C, 16D so as to then be conveyed from the respective channels 15A, 15B, 15C, 15D towards i passages 21A and 21B and flow out through the outlet connector 10.

Thanks to the arrangement described above, and in particular the direct communication of the intermediate interspace 23B with the external with the vent holes 11A, 11B, 11C and 11D through the hydrophobic membranes 24B, 24C, which also allows the liquid in such intermediate interspace to be deaerated effectively, the filtering capacity of the filter according to the invention is significantly improved.

The filter 1 according to the invention is normally used in a vertical portion of an infusion line, i.e. it is oriented so that the inlet connector 9 is positioned at the upper part with respect to the outlet connector 10. After touching the proximal hydrophobic membranes 24A, 24B, 24C, 24D should the liquid still contain air, for example due to an orientation of use different from the one described above, the residual air can flow out through the distal membranes 24A, 24B, 24C, 24D and the respective manifolds 22A, 22B, 22C, 22D connected respectively the holes 11C and 11D.

The presence of the four aeration holes 11A,11B,11C,11D and the arrangement of the hydrophobic membranes 24A,24B,24C,24D upstream and downstream of the flow in the interspaces 23A,23B,23C,23D of the filter 1 reveals the further advantage lying in the fact that, in particular during the priming, the air contained in the liquid accumulates in the end areas of the filter 1 so as not to occlude part of the filtering surfaces; in this manner, the aeration capacity of the filter is increased further with respect to the prior art solutions described above.

Obviously, the construction details and the embodiments may widely vary with respect to what has been described and illustrated, without departing from the scope of protection of the present invention as defined in the claims that follow.

Therefore, although the embodiment described with reference to the drawings provides for four hydrophilic membranes, even solutions in which - within the filter - there is provided for a different number of membranes, for example three or five or more, fall within the scope of protection of the present invention.

## Claims

1. Filter (1) for infusion medical lines comprising a box-like body (2) formed by a first (5) and by a second (6) half-shell and provided with an inlet connector (9) for a liquid to be filtered and with an outlet connector (10) for the filtered liquid, within the box-like body (2) there being provided for:
- a first plate-like element (13A) on whose opposite faces there are formed ribs (14) defining respective first channels (15A,15B) which extend parallel between said connectors (9, 10), on said ribs (14) of each face of said first plate-like element (13A) there being arranged respectively a first (16A) and a second (16B) filtering hydrophilic membrane which separate said first channels (15A,15B) respectively from a first interspace (23A) delimited by said first half-shell (5) and from a second interspace (23B), said first and second interspace (23A,23B) being placed in communication with the inlet connector (9) and said first interspace (23A) being connected with the external through first vent openings (11A, 11C) with which there are associated respective hydrophobic membranes (24D), said first channels (15A,15B) being placed in communication with the outlet connector (10),
- a second plate-like element (13B) parallel and spaced apart with respect to said first plate-like element (13A) and on whose opposite faces there are formed ribs (14) defining respective second channels (15C,15D) which extend parallel between said connectors (9, 10), wherein on said ribs (14) of each face of said second plate-like element (13B) there are arranged respectively a third (16C)and a fourth (16D) filtering hydrophilic membrane which separate said second channels (15C,15D) respectively from the second interspace (23B) and from a third interspace (23C) delimited by said second half-shell, said third interspace (23C) being placed in communication with the inlet connector (9) and being connected with the external through second vent openings (11B,11D) with associated hydrophobilic membranes (24A), said second channels (15C,15D) being placed in communication with the outlet connector (10),
**characterised in that** said second interspace(23B) is placed in direct communication with the external through said vent openings (11A, 11B, 11C, 11D) and associated hydrophobic membranes (24B,24C).

2. Filter (1) according to claim 1, **characterised in that** at the ends of the opposite faces of each of said plate-like elements (13A,13B) there are provided for respective first and second transverse manifolds (22A,22B,22C,22D) on which there are arranged respectively said first and second hydrophobic membranes (24A,24B,24C,24D), said first transverse manifolds (22A,22B) being placed in communication with said first vent openings (11A and 11C), and said second transverse manifolds (22C,22D) being placed in communication with said second vent openings (11B,11D).

3. Filter according to claim 1 or 2, **characterised in that** said outlet connector (10) comprises a double insert sealingly fixed to the box-like body (2) and forming a first duct (30) placed in communication with said first and second interspace (23A, 23B) through a first passage (21A), and a second duct (31) placed in communication with said second and said third interspace (23B, 23C) through a second passage (21B) therefore the flows of the filtered liquid through said first and said second hydrophylic membranes (16A, 16B) on one side and said third and said fourth hydrophylic membranes (16C, 16D) on the other are separated through said first and second duct (30, 31) until the outflow from the filter.

4. Filter (1) according to one or more of the preceding claims, **characterised in that** said inlet connector (9) and said outlet connector (10) are designed for tube-tube or luer-tube or tube-luer or luer-luer connections of said medical line.

5. Filter (1) according to one or more of the preceding claims, **characterised in that** at least one of said filtering hydrophilic membranes (16A,16B,16C,16D) is made as a single piece with said hydrophobic membranes (24A,24B,24C,24D).
